(19)

Europäisches
Patentamt
European
Patent Office
Office européen
des brevets

(11) **EP 1 927 652 A1**

(12) **EUROPEAN PATENT APPLICATION**

(43) Date of publication:
**04.06.2008 Bulletin 2008/23**

(51) Int Cl.:
**C12M 3/00** (2006.01)

(21) Application number: **06124973.6**

(22) Date of filing: **29.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Koninklijke Philips Electronics N.V.
5621 BA Eindhoven (NL)**

(72) Inventor: **The designation of the inventor has not yet been filed**

(74) Representative: **Van Velzen, Maaike Mathilde
Philips
Intellectual Property & Standards
P.O. Box 220
5600 AE Eindhoven (NL)**

(54) **Cell array or matrix assembly and electroporation**

(57)     The present invention relates to an apparatus 1 for transferring cells 2 suspended in a fluid 3 from the fluid 3 to a substrate 17. The apparatus 1 comprises a transfer device 8 having an exterior surface 9, an interior cavity 10, and a plurality of passages 11 each having a first end 12 at the exterior surface 9 and a second end 13 in fluid communication with the cavity 10 and each having a diameter corresponding to a predefined size of the cells 2 to be transferred. The apparatus further comprises a pressure regulating device 14 capable of regulating a pressure in the cavity 10 so as to establish a pressure difference between the first and second ends 12, 13 of each passage 11, a container 4 for containing the fluid 3, and a flow device 7 capable of causing the fluid 3 to flow across the exterior surface 9 when in contact with the fluid 3.

By appropriate selection of passage diameter, pressure difference and fluid flow velocity, the transfer device can be used to pick up and transfer cells 2 within a predefined size range. The invention further relates to a method for transferring cells 2 by use of such an apparatus 1.

Fig. 1

**Description**

FIELD OF THE INVENTION

The present invention relates to cell array and matrix assembly and in particular to transfer of cells suspended in a fluid from the fluid to a substrate.

BACKGROUND OF THE INVENTION

An important step in research studies in the field of gene therapy and drugs discovery is the disposition of cells in an array or a matrix on a substrate. This is typically done by preparing a sample containing a certain concentration of cells in a liquid and subsequently dispensing from a needle. To ensure a desired cell size, filtration may be a step in the preparation of the sample. One of the limitations of the technology is the speed with which the array or matrix can be build up. Another limitation is that it is difficult to dispense cells individually from a suspension in a reliable way. In patch clamp technology an array of sub-cellular sized holes in a glass plate can be used for attaching cells by under-pressure; see e.g. Fertig et al., Biophysics Journal, June 2002, 82(6), p. 3056.

Studies within the field of gene therapy and drugs discovery often involve electroporation used for introduction of a substance, such as e.g. DNA, into the cells. This is typically done by deposing a droplet of the substance to each cell and applying an electrical field that temporarily increases the permeability of the cell membrane.

Hence, a more efficient technique for transferring cells from a fluid to a substrate would be advantageous, and in particular a technique that renders preceding filtration of the cells superfluous would be advantageous.

SUMMARY OF THE INVENTION

Accordingly, the invention preferably seeks to mitigate, alleviate or eliminate one or more of the above mentioned disadvantages singly or in any combination. In particular, it may be seen as an object of the present invention to improve the efficiency with which cells can be transferred from a fluid in which they are suspended to form an array or a matrix of cells on a substrate. It may be seen as a further object of the present invention to enable transfer of cells within a predefined size range. These objects and several other objects are obtained in a first aspect of the invention by providing an apparatus for transferring cells suspended in a fluid from the fluid to a substrate, the apparatus comprising a container for containing the fluid and a transfer device having an exterior surface, an interior cavity, and a plurality of passages each having a first end at the exterior surface and a second end in fluid communication with the cavity and each having a diameter corresponding to a predefined size of the cells to be transferred, and the apparatus further comprising a pressure regulating device capable of regulating a pressure in the cavity so as to establish a pressure difference between the first and second ends of each passage, and a flow device capable of causing the fluid to flow across the exterior surface when in contact with the fluid.

By appropriate selection of passage diameter, pressure difference and fluid flow velocity, the transfer device can be used to pick up cells within a predefined size range. Cells that are smaller than the diameter of the passages are sucked up through the passages, and very large cells are not retained against the first ends of the passages due to the too large fluid drag on these cells. Therefore, by use of an apparatus according to the present invention, complicated sample preparation steps to select the target cell type from biological samples can be simplified or even omitted. Furthermore, cells are handled individually so that the cell array or matrix on the substrate to which the cells are transferred has only one cell at each separate position. The transfer device may e.g. correspond to an ink jet head as used in a printer, or it may be a flat substrate with an array of nozzles that can be pressurized at one side.

An apparatus according to the present invention may comprise a detector for detecting when passages are blocked by a cell, and a controller for causing the transfer device to move to the second position in which the pressure difference is controllable so that the cells will be released from the passages and attach to the substrate. It may e.g. be required that a predefined fraction of the passages are blocked, before the cells are transferred.

The velocity V of the fluid in the container near the average position of an attached cell is preferably in the range of $0.1 \, V_{char} < V < 10 \, V_{char}$, where the characteristic velocity is:

$$V_{char} = D_{passage} \cdot \Delta p \, / \, 12\eta$$

$D_{passage}$ is the diameter of the passages, $\Delta p$ is the pressure difference between fluid in the container and in the cavity, and $\eta$ is the dynamic velocity of the fluid. However, any other relationship between the parameters and any velocity range is covered by the scope of the invention.

The transfer device may also be useable for disposal of substance containing one or more materials that is/are to be brought into one or more cells on transferred cells. Such a substance may e.g. comprise DNA, antisense agents or pharmaceuticals under investigation in the field of gene therapy or drugs discovery.

In an embodiment of the invention, the transfer device further comprises electrodes whereby a potential can be applied to transferred cells. This may e.g. be used to perform electroporation and transfection, but any purpose of applying a potential will be possible within the scope of the invention. The electrodes may form a pattern that enables the potential to be applied to perform selective electroporation of one or more cells. Hereby it will e.g. be possible to study the effect of transfection of selected cells only or to perform the electroporation at certain time intervals to study time dependent effects.

Another aspect of the present invention is provided by a method for transferring cells from a fluid to a substrate using an apparatus as described above. The method comprises the steps of establishing contact between the fluid and the exterior surface, establishing the pressure difference between the first and second ends of each passage, establishing fluid flow across the exterior surface so that cells larger than the predefined size are not retained against the first ends of the passages, bringing the first ends of each passage and the substrate in close proximity of each other, and varying the pressure difference so that the cells attach to the substrate.

In embodiments of the invention in which electroporation can be performed by application of a potential, the electrodes may be placed on the substrate, and as described for the transfer device, these electrodes may form a pattern that enables selective electroporation of one or more cells. It is also possible within the scope of the invention to have electrodes both on the transfer device and on the substrate.

BRIEF DESCRIPTION OF THE DRAWINGS

The present invention will now be explained, by way of example only, with reference to the accompanying figures, where

Figure 1 is a schematic illustration of a transfer device picking up cells suspended in a fluid;
Figure 2 is a schematic illustration of a part of a transfer device releasing cells so that they form a matrix on a substrate; and
Figure 3 is a schematic illustration of disposing of a substance on the cells attached to a substrate.

The drawings in the figures are not to scale.

DETAILED DESCRIPTION OF AN EMBODIMENT

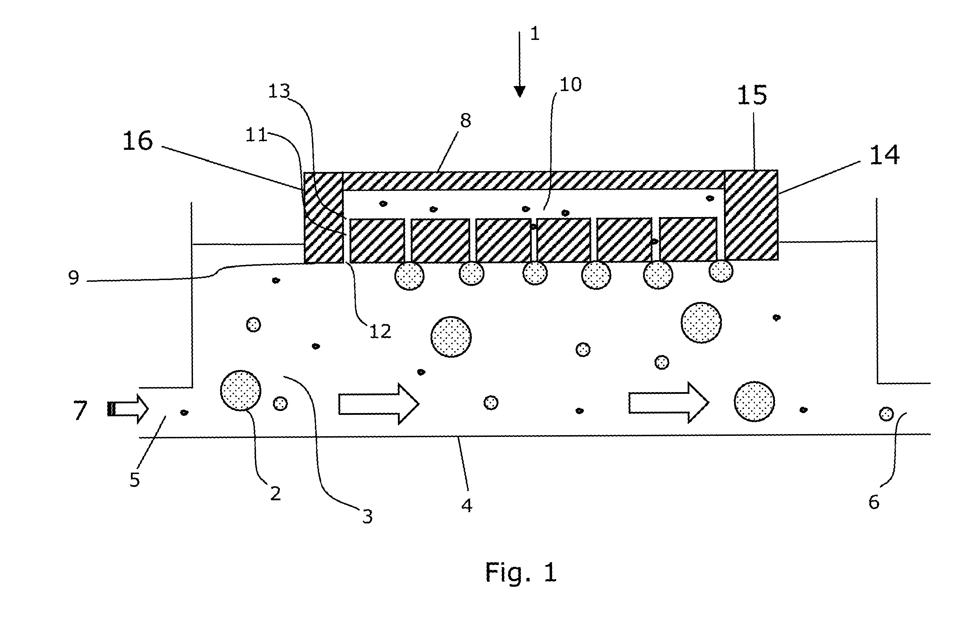

An apparatus 1 according to the present invention enables building up an array or a matrix of cells 2 from a biological sample. The cells 2 are initially suspended in a fluid 3 that is filled in a container 4 having an inlet 5 and an outlet 6 as illustrated schematically in figure 1. The container 4 is connected to a flow device 7 capable of establishing a flow of fluid 3; such a flow device 7 may e.g. be an electrical pump. In an embodiment of the invention, the fluid 3 is re-circulated to the container 4, whereby the total amount of fluid 3 and cells 2 can be kept to a minimum. The figure shows schematically a transfer device 8 having an exterior surface 9, an interior cavity 10 and a plurality of passages 11. The passages 11 have a first end 12 at the exterior surface 9 and a second end 13 in fluid communication with the cavity 10.
The apparatus 1 comprises a pressure regulating device 14 by use of which a pressure difference can be established between the first and second ends 12, 13 of each passage 11 and thereby between the cavity 10 and the fluid 3 in the container 4. When the exterior surface 9 of the transfer device 8 is in contact with the fluid 3, the pressure difference causes the fluid 3 to flow into the cavity 10 via the passages 11. Hereby cells 2 that are smaller than the diameter of the passages 11 can be sucked up and led away via the cavity 10, whereas larger cells 2 are retained against the first ends 12 of the passages 11. From the cavity 10 the fluid 3 may be led to a second container (not shown). In the situation illustrated in figure 1, all but one of the passages 11 are blocked by a cell 2.
The transfer device 8 can be use to pick up cells 2 within a predefined size range. This is obtained by an appropriate choice of the diameter of the passages 11 and by application of a fluid flow in the container 4 as described above. The velocity of the fluid 3 and the pressure difference can be adjusted so that cells 2 that are too big cannot be retained against the first ends 12 of the passages 11 but are washed away via the outlet 6 of the container 4. By use of this embodiment of the invention, it therefore becomes unnecessary to filter the sample of cells 2 in a fluid 3 which simplifies the process. Cells 2 may stick together incidentally. If this happens, they will either be washed away due to their total larger size, or they will be separated by the transversal flow.
Theoretical modeling has been used to determine an appropriate relationship for the choice of parameter settings for a given application. It was found that the following equation can be used. The velocity V of the fluid 3 in the container 4 near the average position of an attached cell 2 should preferably be in the range of 0.1 $V_{char} < V < 10\ V_{char}$, where the characteristic velocity is:

$$V_{char} = D_{passage} \cdot \Delta p\ /\ 12\eta$$

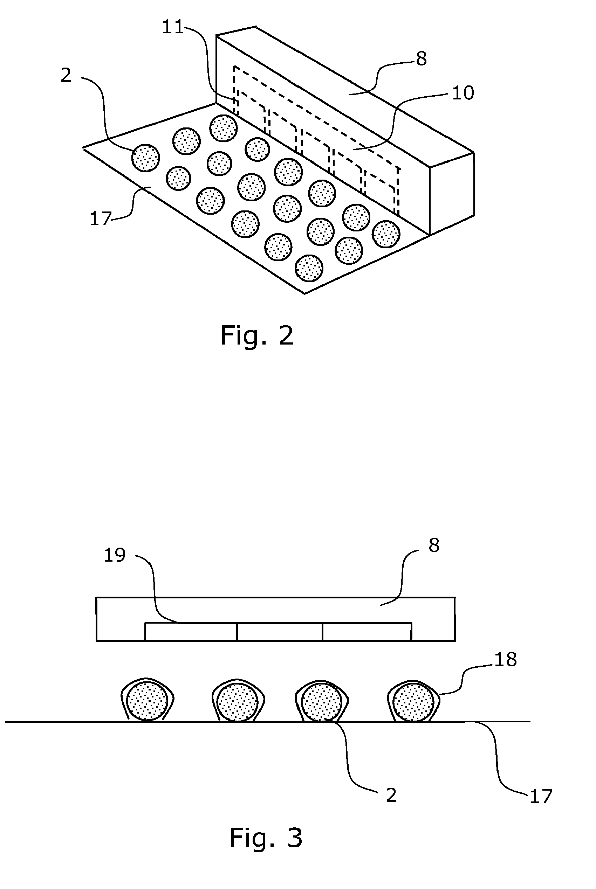

$D_{passage}$ is the diameter of the passages 11, $\Delta p$ is the pressure difference between fluid 3 in the container 4 and in the cavity 10, and $\eta$ is the dynamic velocity of the fluid 3. However, any other relationship between the parameters and any velocity range is covered by the scope of the invention. When a cell 2 is attached to a passage 11, $\Delta p$ is the pressure difference over the cell 2, i.e. the pressure difference between the fluid 3 at the side of the cell 2 facing the container 4 and the fluid 3 at the other side facing the passage 11.
In the embodiment shown in figure 1, a detector 15 detects when all or a predefined fraction of the passages 11 are blocked by a cell 2. Subsequently a controller 16 causes the transfer device 8 to move to a position in close proximity of the substrate 17 (see figure 2) to which the cells 2 are to attach for further treatment. The detection may e.g. be related to a decreased fluid flow leaving the cavity 10 when more and more passages 11 are blocked. It may alternatively be related to the pressure regulation needed to maintain the pressure difference.

Figure 2 shows schematically how a cell 2 matrix can be build up by transferring one row of cells 2 at a time to the substrate 17. In another (not shown) embodiment of the invention, the transfer device 8 comprises more than one row of passages 11 so that more rows of cells 2 can be transferred at a time. The cells 2 are released from the transfer device 8 by removing the pressure difference, i.e. the under-pressure in the cavity 10, and if necessary by applying a slight over-pressure.

An alternative to moving the transfer device 8 is to keep it in a fixed position and to move the container 4 and the substrate 17.

If desired, it may be possible to use different flow velocities for different rows. Hereby it is possible to vary the upper limit of the size range of cells 2 between the rows. This may e.g. be relevant if influence from the cell size, or another parameter directly related thereto, is a variable under investigation in a given research study.

In an (not shown) embodiment of the invention, the transfer device 8 is oriented so that the first end 12 of the passages 11 point upwards before the cells 2 are to be released. Hereby it can be ensured that possible fluid 3 remains are not leaving the passages 11.

In an embodiment of the invention, the transfer device 8 is used not only to build up an array or a matrix of cells 2 as described above but also to perform subsequent electroporation and transfection. After the array or matrix has been built up, the passages 11 and the cavity 10 of the transfer device 8 are emptied, cleaned and filled with DNA, antisense agents, pharmaceuticals or another substance 18 that is to be brought into one or more cells 2. The transfer device 8 is then moved to a position in which the first ends 12 of the passages 11 are in close proximity of the cells 2 on the substrate 17, and the substance 18 is disposed on one or more of the cells 2. Figure 3 is a schematic illustration of the disposing of a substance 18 on the cells 2. The substrate 17 should preferably be hydrophobic, such as having a contact angle larger than 50°. This will keep the disposed substance 18 close to the cells 2 so that the thickness of the substance 18 layer is well defined.

The electroporation can subsequently be performed by applying an electrical potential to the cells 2. The potential can be applied via electrodes 19 on either the transfer device 8 or the substrate 17 or on both. Figure 3 shows an example of the placement of electrodes 19 on the transfer device 8. The potential will typically be of the order from a few volts to several hundreds of volts depending on the distance between the transfer device 8 and the substrate 17. Depending on the electrode pattern, single- or multi-cell electroporation can be performed. The application of a potential may also be used to draw droplets of substance 18 out of the passages 11.

An alternative to using the same transfer device 8 for transfer of both cells and substance 18 is to use one or more other transfer devices (not shown) for disposing substance 18 than the one used to transfer the cells 2. This alternative may increase the working speed, minimize the waste of fluid 3 and substance 18, and minimize the risk of contamination due to undesired mixing of remains of fluid 3 and substances 18. Such other transfer devices may have a design different from the one used to transfer the cells 2, as they may not comprise e.g. the cavity 10 and the pressure regulating device 14.

In all embodiments described above it is typically important to ensure that the cells 2 are attached to the substrate 17 at predefined positions, and that the substance 18 comprising material to be brought into the cells 2 is disposed precisely on the cells 2. The choice of appropriate alignment technology for this purpose will be obvious for a person skilled in the art.

The passages 11 may be divided into groups each connected to a separate cavity (not shown) so that one or more groups of cells 2 can be released at a time. Hereby it will e.g. be possible to place the cells 2 on the substrate 17 in a pattern different from the one formed by the position of the passages 11 of the transfer device 8. This possibility may alternatively be obtained by use of a piezo-facility known e.g. from an ink-jet head of an ink-jet printer. These options may also be used to apply substance 18 to some of the cells 2 only.

Although the present invention has been described in connection with the specified embodiments, it is not intended to be limited to the specific form set forth herein. Rather, the scope of the present invention is limited only by the accompanying claims. In the claims, the term "comprising" does not exclude the presence of other elements or steps. Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

**Claims**

1.  An apparatus (1) for transferring cells (2) suspended in a fluid (3) from the fluid (3) to a substrate (17), the apparatus (1) comprising

    - a container (4) for containing the fluid (3),
    - a transfer device (8) having an exterior surface (9), an interior cavity (10), and a plurality of passages (11) each having a first end (12) at the exterior surface (9) and a second end (13) in fluid communication with the cavity (10) and each having a diameter corresponding to a predefined size of the cells (2) to be transferred,
- a pressure regulating device (14) capable of regulating a pressure in the cavity (10) so as to establish a pressure difference between the first and second ends (12, 13) of each passage (11), and
- a flow device (7) capable of causing the fluid (3) to flow across the exterior surface (9) when in contact with the fluid (3).

2. An apparatus (1) according to claim 1, in which the transfer device (8) has

- a first position in which the first ends (12) of the passages (11) are in fluid communication with the fluid (3) in the container (4), and in which at least one of the pressure difference or the fluid flow across the exterior surface (9) is controllable to retain cells (2) having the predefined size against the first ends (12) of the passages (11), and
- a second position in which the pressure difference is controllable so that the cells (2) will be released from the passages (11) and attach to the substrate (17).

3. An apparatus (1) according to claim 2, further comprising

- a detector (15) for detecting when passages (11) are blocked by a cell (2), and
- a controller (16) for causing the transfer device (8) to move to the second position.

4. An apparatus (1) according to claim 1, wherein the velocity V of the fluid (3) in the container (4) near the average position of an attached cell (2) is in the range of $0.1 \, V_{char} < V < 10 \, V_{char}$,
where the characteristic velocity is:

$$V_{char} = D_{passage} \cdot \Delta p \, / \, 12\eta$$

$D_{passage}$ is the diameter of the passages (11), $\Delta p$ is the pressure difference between fluid (3) in the container (4) and in the cavity (10), and $\eta$ is the dynamic velocity of the fluid (3).

5. An apparatus (1) according to claim 1, wherein the transfer device (8) can be oriented so that the first ends (12) of the passages (11) point upwards before the cells (2) are attached to the substrate (17).

6. An apparatus (1) according to claim 1, wherein the pressure difference between the first and second ends (12, 13) of each passage (11) can be controlled individually or in groups, so as to release cells (2) selectively.

7. An apparatus (1) according to claim 1, wherein the transfer device (8) can also be used to dispose a substance (18) containing one or more materials that is/are to be brought into one or more cells (2) on transferred cells (2).

8. An apparatus (1) according to claim 1, wherein the transfer device (8) further comprises electrodes (19) whereby a potential can be applied to transferred cells (2).

9. An apparatus (1) according to claim 8, wherein the electrodes (19) form a pattern that enables the potential to be applied to perform selective electroporation of one or more cells (2).

10. A method for transferring cells (2) from a fluid (3) to a substrate (17) using an apparatus (1) according to claim 1, the method comprising the steps of

- establishing contact between the fluid (3) and the exterior surface (9),
- establishing the pressure difference between the first and second ends (12, 13) of each passage (11),
- establishing fluid flow across the exterior surface (9) so that cells (2) larger than the predefined size are not retained against the first ends (12) of the passages (11),
- bringing the first ends (12) of the passages (11) and the substrate (17) in close proximity of each other, and
- varying the pressure difference so that the cells (2) attach to the substrate (17).

11. A method according to claim 10, further comprising the steps of:

- emptying and cleaning the passages (11) and the cavity (10) of the transfer device (8),
- filling the passages (11) and the cavity (10) with a substance (18) containing one or more materials that is/are to be brought into one or more cells (2),
- moving the substrate (17) or the transfer device (8) to a position in which the first ends (12) of the passages (11) are in close proximity of the cells (2) on the substrate (17),
- disposing the substance (18) on one or more cells (2), and
- applying an electrical potential to electrodes (19) to perform electroporation.

12. A method according to claim 10, further comprising the steps of

- moving the substrate (17) or one or more additional transfer devices comprising passages (11) containing a substance (18) comprising one or more materials that is/are to be brought into one or more cells (2) to a position in which the passages (11) are in close proximity of the cells (2) on the substrate (17),
- disposing the substance (18) on one or more cells (2), and
- applying an electrical potential to electrodes (19) to perform electroporation.

13. A method according to claim 11 or 12, wherein the electrodes (19) are placed on the substrate (17).

14. A method according to claim 11 or 12, wherein the electrodes (19) form a pattern that enables selective electroporation of one or more cells (2).

Fig. 1

EP 1 927 652 A1

Fig. 2

Fig. 3

**European Patent Office**

**EUROPEAN SEARCH REPORT**

Application Number

EP 06 12 4973

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 05 076341 A (TOKIMEC INC) 30 March 1993 (1993-03-30) * abstract * ----- | 1,2,4-7, 10 | INV. C12M3/00 |
| X | EP 1 707 620 A (FUJITSU LTD [JP]) 4 October 2006 (2006-10-04) * paragraphs [0012], [0019], [0021], [0025], [0027], [0054] * ----- | 1-7,10 | |
| A | EP 0 962 524 A1 (MICRONAS INTERMETALL GMBH [DE] MICRONAS GMBH [DE]) 8 December 1999 (1999-12-08) * paragraphs [0008], [0015], [0026] * * claims; figures * ----- | 1,7-14 | |
| A | EP 1 571 201 A2 (MITSUTECH CO LTD [JP]; GENE MEDICINE JAPAN INC [JP]) 7 September 2005 (2005-09-07) * paragraphs [0008], [0009] * * claims * ----- | 1,10 | |
| A | WO 2004/074426 A2 (FRAUNHOFER GES FORSCHUNG [DE]; FUHR GUENTER R [DE]; ZIMMERMANN HEIKO []) 2 September 2004 (2004-09-02) * claims; figures * ----- | 1,10 | TECHNICAL FIELDS SEARCHED (IPC) C12M C12N |
| A | US 5 902 745 A (BUTLER MARK D [US] ET AL) 11 May 1999 (1999-05-11) * column 1, lines 7-9 * * column 3, lines 17-49 * * column 4, lines 17-20 * * column 5, lines 16-48 * * claims; figures * ----- | 1-14 | |
| A | EP 1 621 912 A (FUJITSU LTD [JP]) 1 February 2006 (2006-02-01) * the whole document * ----- | 1-3,7 | |

The present search report has been drawn up for all claims

| Place of search | Date of completion of the search | Examiner |
|---|---|---|
| Berlin | 8 March 2007 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 12 4973

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2007

| Patent document cited in search report | | Publication date | Patent family member(s) | | Publication date |
|---|---|---|---|---|---|
| JP 5076341 | A | 30-03-1993 | NONE | | |
| EP 1707620 | A | 04-10-2006 | JP | 2006280231 A | 19-10-2006 |
| | | | US | 2006223163 A1 | 05-10-2006 |
| EP 0962524 | A1 | 08-12-1999 | JP | 11346764 A | 21-12-1999 |
| | | | US | 6475760 B1 | 05-11-2002 |
| EP 1571201 | A2 | 07-09-2005 | JP | 2005218413 A | 18-08-2005 |
| | | | US | 2005176138 A1 | 11-08-2005 |
| WO 2004074426 | A2 | 02-09-2004 | AU | 2003283436 A1 | 09-09-2004 |
| | | | AU | 2003289944 A1 | 09-09-2004 |
| | | | AU | 2003294766 A1 | 09-09-2004 |
| | | | DE | 10307487 A1 | 09-09-2004 |
| | | | WO | 2004074425 A2 | 02-09-2004 |
| | | | WO | 2004074424 A2 | 02-09-2004 |
| | | | EP | 1594948 A2 | 16-11-2005 |
| | | | EP | 1594947 A2 | 16-11-2005 |
| | | | EP | 1594949 A2 | 16-11-2005 |
| | | | KR | 20050105479 A | 04-11-2005 |
| | | | KR | 20050095655 A | 29-09-2005 |
| | | | KR | 20050105480 A | 04-11-2005 |
| | | | US | 2006051735 A1 | 09-03-2006 |
| | | | US | 2006194309 A1 | 31-08-2006 |
| | | | US | 2006134600 A1 | 22-06-2006 |
| US 5902745 | A | 11-05-1999 | AU | 6854696 A | 09-04-1997 |
| | | | FR | 2739028 A1 | 28-03-1997 |
| | | | IT | MI961913 A1 | 18-03-1998 |
| | | | NL | 1004082 C2 | 10-04-1998 |
| | | | NL | 1004082 A1 | 25-03-1997 |
| | | | WO | 9710807 A1 | 27-03-1997 |
| EP 1621912 | A | 01-02-2006 | JP | 2006034174 A | 09-02-2006 |
| | | | US | 2006024812 A1 | 02-02-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FERTIG et al.** *Biophysics Journal,* June 2002, vol. 82 (6), 3056